# EUROPEAN PATENT APPLICATION

(11) **EP 4 233 815 A1**
(43) Date of publication of application: **30.08.2023**
(21) Application number: 22382152.1
(22) Date of filing: 23.02.2022
(51) Int. Cl.: A61F 13/08, A41D 13/00, A41D 1/21, A41B 11/00, A41D 31/18

(54) **MULTIDIRECTIONAL COMPRESSION GARMENT**

(71) Applicant: Nueva Expression Textil SA, 28010 Madrid (ES)
(72) Inventor: DIAS, Hernâni, 28010 Madrid (ES)
(74) Representative: Clarke, Modet y Cía., S.L.

(57) **Abstract**

The present invention relates to a multidirectional compression garment. The multidirectional compression garment (1) comprising a compression material made of a seamless knitted fabric, wherein the fabric has at least one of the following structures: a first structure (2) having a first pattern such that the first pattern comprises identical parallel columns of fibers with a gap between each two adjacent columns; a second structure (3) having a second pattern such that the second pattern comprises parallel columns of fibers with a gap between each two adjacent columns; and a third structure (4) having a third pattern such that the third pattern comprises parallel columns of a first type of fibers (5) and a second type of fibers (6). The multidirectional compression garment (1) provides different levels of compression depending on the area of the body, with adequate elasticity, which is comfortable for the user, and which has beneficial effects on the user's health.

## Description

### FIELD OF THE INVENTION

The present invention relates to a multidirectional compression garment. In particular, the present invention relates to a compression material made of a seamless knitted fabric, wherein the fabric has different structures.

### BACKGROUND ART

The presence of compression in textile articles can be defined as the decrease in intrinsic thickness, with a corresponding increase in pressure [1]. Chronic venous disease management, scar management, orthopaedic support, sports and body shaping are some of the main application areas for this type of articles [2].

Compression garments have become very popular among athletes. The compression areas usually vary from full limbs to limb segments (socks, t-shirts, tights, shorts, and sleeves). The compression garment is generally produced from a mixture of fibers, with greater or lesser amounts of elastane, being the elasticity of the two materials present one of the two great influencers in the changes of applied pressure on fabrics and muscles [3].

The elastomeric fibers possess properties that are not found in nature, such as high elasticity. These fibers show maximum force extension greater than 200%, rapid recovery, with a high extension reversibility after 400%-800% elongation. Spandex^{®}, Lycra^{®} and Dorlastan^{®} are the most commercially known fibers [6].

Compression garments are typically smaller than the body site to be covered, and compression is obtained by the elongation force exerted on the fibers and yarns. The elasticity of the materials used has a great influence on the changes in the pressure applied to the tissue and the muscle pump function; it was shown that inelastic compression on the calf led to a significant increase in venous pumping, while elastic sport stockings did not show any significant change [4].

Despite the deep study carried out on the compression mechanisms, these are still not fully understood, because the way in which human tissues respond to external forces is partially unknown [3].

Furthermore, and regardless of the location or purpose of application, the compression is applied in specific areas, whether for operational or medical reasons. The evaluation of the press of a textile is denoted as two main factors in the evaluation of comfort, function and safety of the compression garment, because an inadequate compression mechanism may have an effect on the energy, efficiency and health of the user. In this sense, if in some way insufficient pressure results in occurrences of limitation of efficiency and reduction of aesthetic appearance, not the opposite, excessive pressure promotes a reduction in the functioning of the heart and lungs, leading to deterioration of the state of health [5].

Multidirectional compression is characterised by the promotion of pressure in different areas of the human body, defined according to the needs of the area of the body in which they are located. The main objective of this technology is to promote blood circulation, maximising the flow of oxygen to the tissues of the human body and the elimination of metabolites present in the bloodstream. Thus, with this type of technology, by modelling the correct compression pattern along the human body, it is possible, on the one hand, to maximise blood flow during sports practice, thus increasing performance and reducing recovery time and possible injuries, and, on the other hand, to adjust blood circulation, preventing or treating problems such as poor blood circulation or venous thrombosis.

For example, EP3135141A1 relates to sports garments, specifically variable compression garments worn by athletes during/after training and/or competition which comprises greater amounts of elastomer used to create greater amounts of compressive force. The total compressive force applied at any particular location by the garment is the sum of the compressive force provided by the elastomer and the compressive force applied by the base textile.

In addition to the aforementioned areas, compression garments also show great viability in the maternity market, as they help correct posture, reduce pressure in the pelvic region caused by the baby, as well as helping the human body (back and abdomen) to adapt to pregnancy and reduce swelling by favouring the return of venous blood to the heart.

One example of this application can be found in US2016106158A1, which discloses a maternity exercise garment for pregnant women includes abdominal and breast support portions integrated with a cover portion. The integrated support has a two-piece abdomen support (abdomen compression fabric and support elastic) to assist in bearing the weight of the pregnant abdomen, thus alleviating the downward pressure associated with body movement and especially with exercise.

Other example is US2022015462A1 which describes a maternity garment, or bodysuit, configured to provide support and comfort to a pregnant woman. The garment may include an opening in the front of the garment exposing the stomach, or belly, of a woman, and include reinforced or compression material that passes underneath the belly providing support to the belly.

In summary, when designing compression garments, there are several important points to consider, such as the type of fibre and elastomeric properties, the recovery of the material after stretching and the greater or lesser compression depending on the area of the user's body.

However, the vast majority of products that exist as compression products are actually constricting products, due to the correct interpretation of the differences between compression and tightening. Products marketed for sports, shapewear, etc. compress the body without this force being studied/controlled, do not positively support muscle activity and do not actively adjust to anatomical changes during physical activity.

Tight clothing causes problems such as: (1) moisture retention (creating the ideal environment for fungal infections and skin irritations), (2) nerve and tendon compression, (3) "meralgia paresthetica" condition, (4) extra tension in the abdomen that enhances gastric reflux, and an (5) increased discomfort caused by irritable bowel syndrome and urinary incontinence.

Overly tight garments counteract improved blood circulation, waste elimination, and reduce power (increase the amount of force the muscles must perform), precisely what a compression garment is intended to increase.

Therefore, there is a need for compression products that avoid excessive and unnecessary pressure and focus on providing a positive benefit to the body through controlled compression.

### SUMMARY OF INVENTION

In view of the problems of the prior art, the main object of the present invention is to provide a multidirectional compression garment that provides different levels of compression depending on the area of the body, with adequate elasticity, which is comfortable for the user, and which has beneficial effects on the user's health.

In a first aspect, the present invention refers to a multidirectional compression garment comprising a compression material made of a seamless knitted fabric, wherein the fabric has at least one of the following structures:
- a first structure having a first pattern such that the first pattern comprises identical parallel columns of fibers with a gap between each two adjacent columns,
- a second structure having a second pattern such that the second pattern comprises parallel columns of fibers with a gap between each two adjacent columns,
- a third structure having a third pattern such that the third pattern comprises parallel columns of a first type of fibers and a second type of fibers.

The first type of fibers is polyamide and the second type is elastane coated with polyamide.

The applications of these multidirectional compression garments are sport garments and maternity garments.

In sport garments, specifically running garments, it is important to consider two essential factors: the main muscles involved in the running movement and the creation of a compression gradient. The advantages of strength, stretchability, tear resistance and elasticity of the multidirectional compression garment make it suitable for constructing a variety of garments useful for a multitude of sport activities.

In the present invention, running garments, specifically tights, the fabric has a first structure located at the ankle and the side of the garment to enhance blood flow vertically - from the area furthest from the heart (the feet) to the heart; a second structure located in the knees to stabilise the knee/ankle to mitigate sprains and micro-injuries, while a third structure is in the calf and thigh area to help the muscles work.

The first and second structure supports the muscle action and blood drive, while the second structure is used for optimal compression for stabilisation/support.

On the other hand, with regard to maternity garments, it is important to consider that the purpose of the application is to increase the comfort and well-being of women. Therefore, garment will have the main function of providing support to key areas such as the lower back, pelvic and breast.

In the case of maternity garments, the third structure is used to support the weight of the belly. In this type of product, compression is applied in a different way, as the structures help to support the extra weight. This third structure dissipates the weight of the belly through the lower back and the rest of the back. For the breast support, a third structure is also used which helps to support the weight of the breast in the same way as the weight of the belly is supported. In this case, the user gets more belly support, lumbar support, weight dispersion over a larger surface area, fatigue reduction and ergonomics.

Garments in accordance with the present invention may comprise tights, sleeves for arms, sleeves for legs, socks, shirts, or any other type of garment that may be worn on the portion of the user anatomy where compression is desired.

### BRIEF DESCRIPTION OF DRAWINGS

The following figures are described below. These illustrate the exemplary embodiments and are not limiting their scope.
Figure 1 shows a first structure of the fabric.
Figure 2 shows a second structure of the fabric.
Figure 3 shows a third structure of the fabric.
Figure 4 shows the frontal view of a tights for running which comprises the first structure on the side and the lowest point, the second structure located in the knee area and the third structure in the thigh and calf area of the user.
Figure 5 shows the back view of a tights for running which comprises the first structure on the side and the lowest point, the second structure located in the knee area and the third structure in the thigh and calf area of the user.
Figure 6 shows the frontal view of a tights for running which comprises the first structure on the side and the lowest point and the third structure located in the thigh, knee and calf area of the user.
Figure 7 shows the back view of a tights for running which comprises the first structure on the side and the lowest point and the third structure located in the thigh, knee and calf area of the user.
Figure 8 shows the frontal view of a shirt for a pregnant user which comprises the third structure located in the area below the breast and belly of the user.
Figure 9 shows the back view of a shirt for a pregnant user which comprises the third structure is located in the back and lumbar area of the user.
Figure 10 shows the lateral view of a shirt for a pregnant user which comprises the third structure located in the area from the shoulders to the armpits, and also the area under the breast of the user.

### DESCRIPTION OF EMBODIMENTS

The multidirectional compression garment (1) of the present invention comprises a compression material made of a seamless knitted fabric, wherein the fabric has different structures.

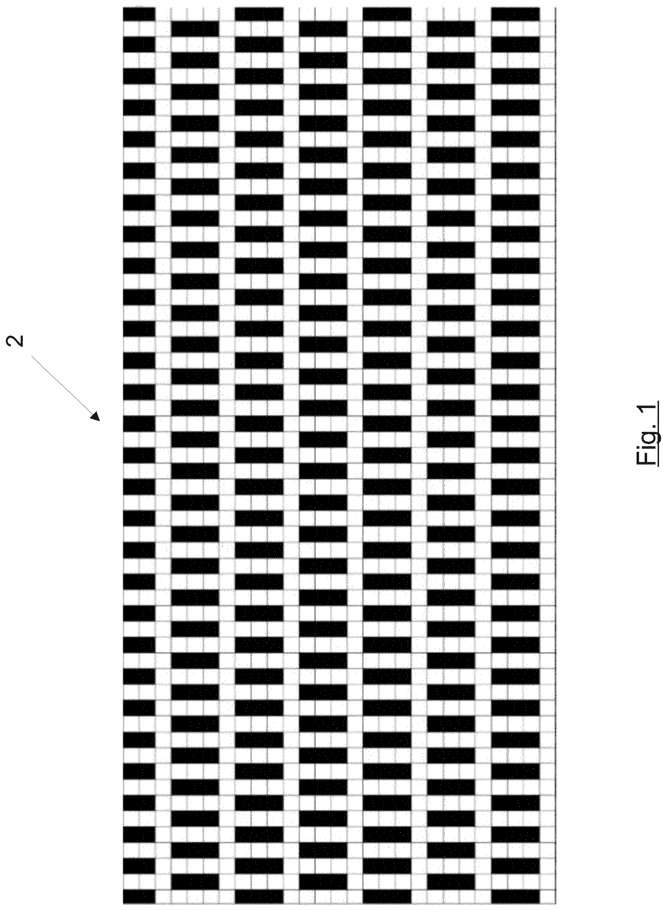

Figure 1 shows the pattern of the first structure (2). This first structure has a first pattern such that the first pattern comprises identical parallel columns of fibers with a gap between each two adjacent columns.

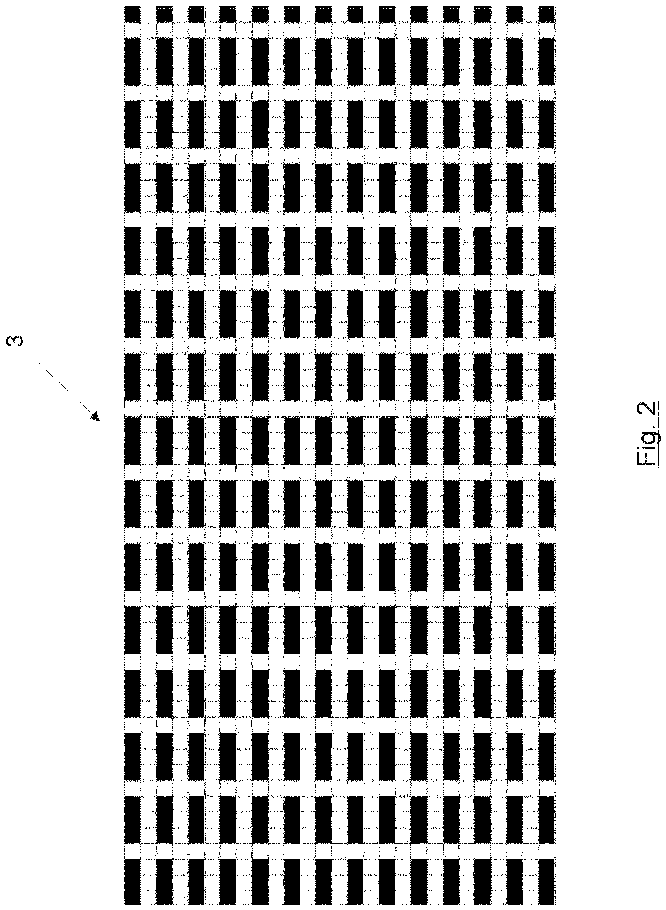

Figure 2 shows the pattern of the second structure (3). This second structure (3) has a second pattern such that the second pattern comprises parallel columns of fibers with a gap between each two adjacent columns.

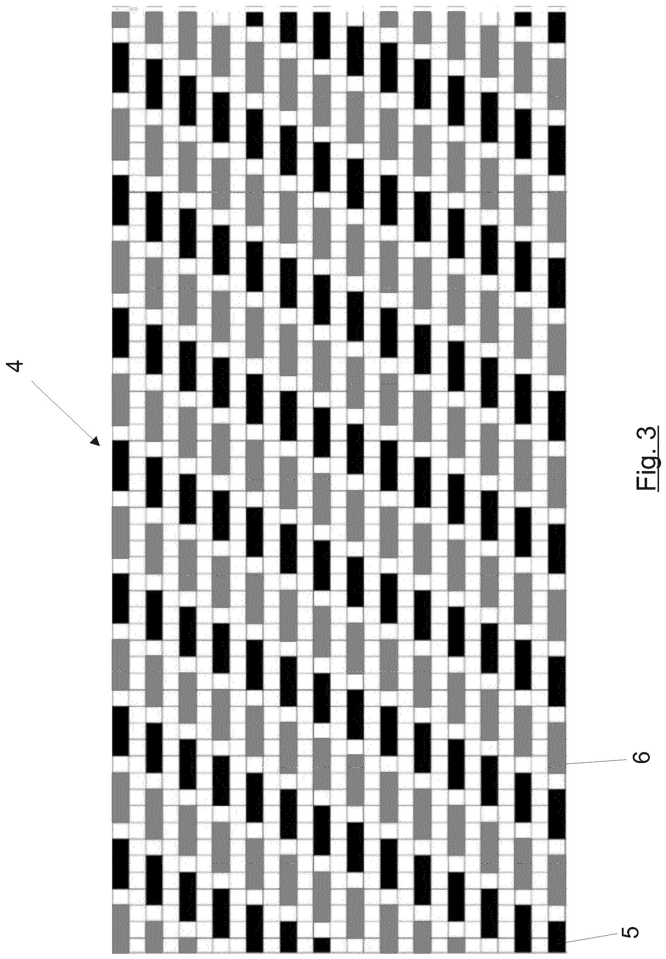

Figure 3 shows the pattern of the third structure (4). This third structure (4) has a third pattern such that the third pattern comprises parallel columns of a first type of fibers (5) and a second type of fibers (6). Preferably, the first type (5) of fibers is polyamide and the second type (6) is elastane coated with polyamide.

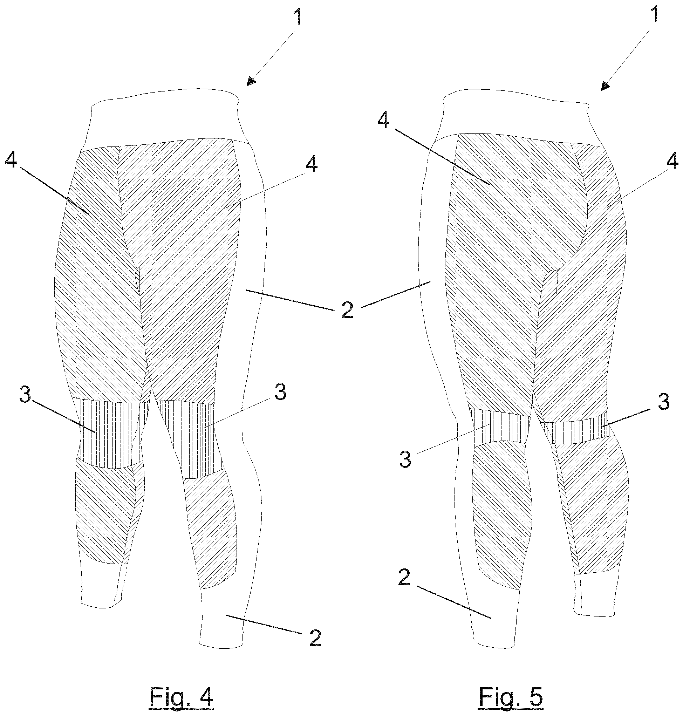

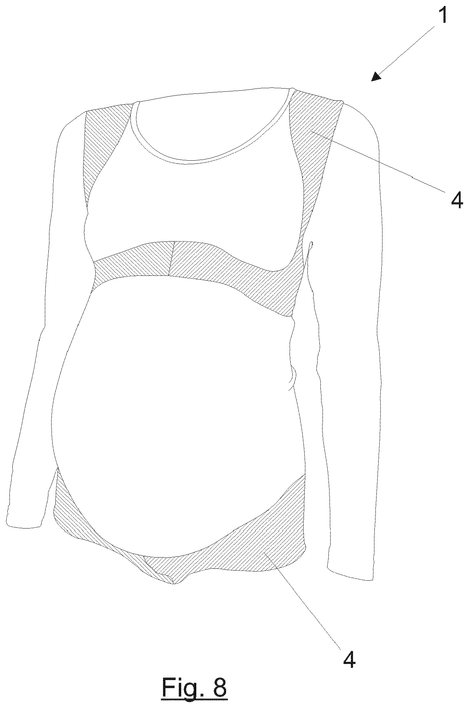

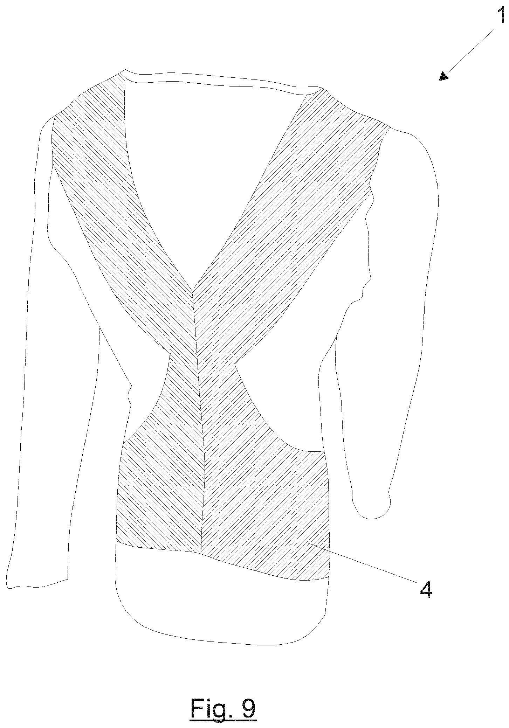

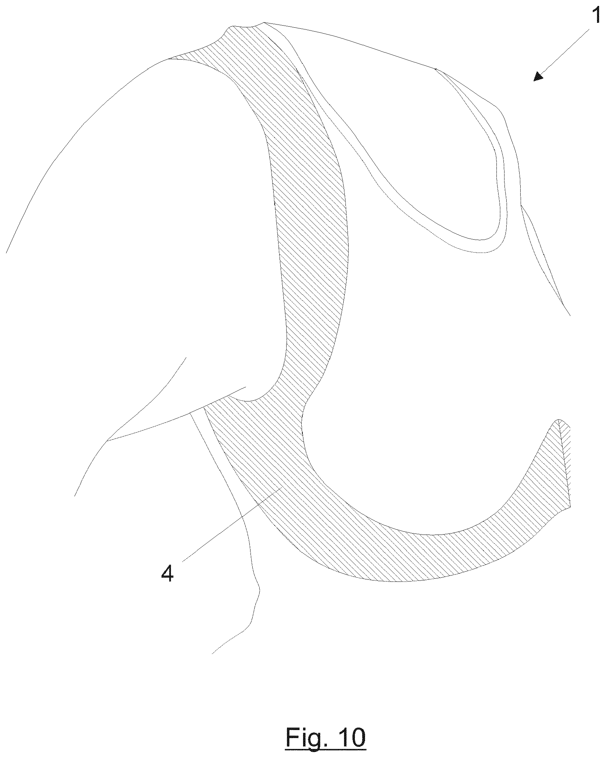

Figures 4-10 illustrates several multidirectional compression garments that can be constructed from the compression material of the present invention. These garments include tights for running (figures 4-7) and a shirt for a pregnant user (figures 8-10).

In a first embodiment (figures 4 and 5), the garment is a sport garment, specifically tights for running. In the garment of this embodiment, the first structure (2) is located on the side and the lowest point, the second structure (3) located in the knee area and the third structure (4) in the thigh and calf area of the user.

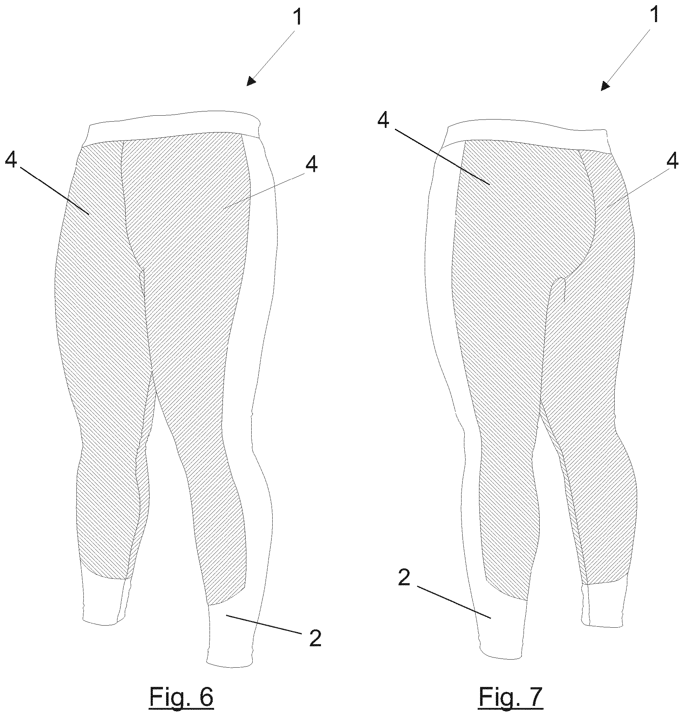

In a second embodiment (figures 6 and 7), the garment is a sport garment but can also be used as a pregnancy garment, specifically tights for running. In the garment of this embodiment, , the first structure (2) is located on the side and the lowest point and the third structure (4) is located in the thigh, knee and calf area of the user.

Thanks to the combination of the different structures and their position in the garment, the following benefits are provided to the user: reduction of fatigue during or after exercise, reduction of muscle vibration during exercise and increased physical performance.

In a third embodiment (figures 8 to 10), the garment is a pregnancy garment, specifically a shirt for a pregnant user. In the garment of this embodiment, the third structure (4) is located in the area below the breast and belly. Furthermore, the third structures (4) is also located in the area from the shoulders to the armpits and in the back and lumbar area.

In this garment, the user gets more belly support, lumbar support, weight dispersion over a larger surface area, fatigue reduction and ergonomics.

### REFERENCES

[1] Murthyguru, "Novel approach to study compression properties in textiles," AUTEX Res. J., vol. 5, no. 4, pp. 176-193, 2005.
[2] Y. Xiong and X. Tao, "Compression Garments for Medical Therapy and Sports," Polymers (Basel)., vol. 10, no. 663, 2018.
[3] Rossi, R. M. "High-performance sportswear." High-Performance Apparel (2018): 341-356.
[4] Mosti, G., and H. Partsch. "Improvement of venous pumping function by double progressive compression stockings: higher pressure over the calf is more important than a graduated pressure profile." European Journal of Vascular and Endovascular Surgery 47.5 (2014): 545-549.
[5] Y.-R. Wang, "Manikins for evaluation of pressure performance," in Manikins for Textile Evaluation, 1st ed., R. Nayak and R. Padhye, Eds. Cambridge: Woodhead Publishing, 2017, pp. 241-258.
[6] M. Senthilkumar, N. Anbumani, and J. Hayavadana, "Elastane fabrics - A tool for stretch applications in sport," Indian J. Fibre Text. Res., vol. 36, pp. 300-307, 2011.

## Claims

1. A multidirectional compression garment (1) comprising a compression material made of a seamless knitted fabric, wherein the fabric has at least one of the following structures:
- a first structure (2) having a first pattern such that the first pattern comprises identical parallel columns of fibers with a gap between each two adjacent columns;
- a second structure (3) having a second pattern such that the second pattern comprises parallel columns of fibers with a gap between each two adjacent columns; and
- a third structure (4) having a third pattern such that the third pattern comprises parallel columns of a first type of fibers (5) and a second type of fibers (6).

2. The multidirectional compression garment (1) according to claim 1, wherein first type of fibers (5) is polyamide and the second type of fibers (6) is elastane coated with polyamide.

3. The multidirectional compression garment (1) according to claim 1 or 2, wherein the garment is a maternity garment.

4. The multidirectional compression garment (1) according to claim 1 or 2, wherein the garment is a sports garment.

5. The multidirectional compression garment (1) according to claim 3, wherein the third structure (4) is located in the area below the breast and belly of the user.

6. The multidirectional compression garment (1) according to claim 3 or 4, wherein , wherein the first structure (2) on the side and the lowest point and the third structure (4) is located in the thigh, knee and calf area of the user.

7. The multidirectional compression garment (1) according to claim 4, wherein the first structure (2) on the side and the lowest point, the second structure (3) located in the knee area and the third structure (4) in the thigh and calf area.
